(19) Europäisches Patentamt
European Patent Office
Office européen des brevets

(11) Publication number: 0 341 793 B1

# (12) EUROPEAN PATENT SPECIFICATION

(45) Date of publication of patent specification: 01.09.93 (51) Int. Cl.5: G01N 27/416

(21) Application number: 89201165.1

(22) Date of filing: 08.05.89

## (54) Buffer solution systems for standardizing ph analyzers and electrolytes.

(30) Priority: 13.05.88 IT 2056088

(43) Date of publication of application:
15.11.89 Bulletin 89/46

(45) Publication of the grant of the patent:
01.09.93 Bulletin 93/35

(84) Designated Contracting States:
BE CH DE ES FR GB LI NL SE

(56) References cited:
EP-A- 0 031 786
DE-A- 3 024 044
DE-A- 3 505 342
US-A- 8 706 343

(73) Proprietor: INSTRUMENTATION LABORATORY S.p.A.
Viale Monza, 338
I-20128 Milano(IT)

(72) Inventor: Manzoni, Angelo
Via U. Foscolo, 22
I-20047 Brugherio (Milano)(IT)
Inventor: Belluati, Mario
Via Pittori Paglia, 13
I-25100 Brescia(IT)

(74) Representative: Faraggiana, Vittorio, Dr. Ing.
Ingg. Guzzi e Ravizza S.r.l. Via Boccaccio, 24
I-20123 Milano (IT)

EP 0 341 793 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to analytical systems for clinical chemistry and more specifically to standardization, standard and/or control solutions used therein for the specific determination of ions (activity or concentration) by direct potentiometry using ion-selective electrodes.

The continuous development of electroactive components for the formulation of ion-selective electrodes of various types enables a vast number of cations and anions to be determined. At present there are at least 30 ion species (anions and cations) which can be determined selectively by direct potentiometry using different types of ion-selective electrodes (Daniel Ammann, Ion-selective microelectrodes, principles, design and application - Springer Verlag, Berlin, Heidelberg, 1986).

Altough glass membrane electrodes are still widely used in analytical chemistry for determining hydrogen ion and sodium ion activity, neutral carrier-based electrodes are gradually assuming significant strategic importance in the determination of alkali and alkaline-earth metals.

In particular, analytical systems for electrolyte determination in the blood, serum and plasma based on direct potentiometry use ion-selective electrodes for sodium, potassium, calcium, lithium and chloride together with the already established pH sensor.

Blood is a complicated biological liquid containing various components of fundamental importance in physiological activity.

An analytical technique which directly indicates the concentration of a substrate in the blood aqueous phase containing both electrolytes and other species such as proteins and lipids either dissolved or present in colloidal form provides fundamental clinical information particularly under conditions of blood abnormality. In this respect, dilution would result in loss of the important information which derives from direct measurement of the intensive properties (concentration) of the system under test.

The indirect method is in fact dependent on the volume of diluting solution, the aqueous part of which will depend on the concentration and types of proteins or lipids present.

The actual ion-selective electrode is an electrochemical half-cell consisting of an internal reference system and the specific membrane, the formulation and function of which depend on the characteristics of the ion to be measured.

The remaining half-cell is an external reference electrode in contact with a standard electrolyte, such as Ag/AgCl in saturated KCl.

The membranes can be of different type according to the ion to be determined.

The potential of the electrochemical cell (electromotive force-EMF, potential difference at zero current) arises when the membrane electrode and reference electrode are both in contact with the solution to be measured.

The electromotive force is the sum of a series of local potential differences generated at the solid-solid, solid-liquid and liquid-liquid interface.

Ideally, the potential difference generated between the membrane and solution to be measured depends on the activity of the types of ion in the sample.

If all other potential differences are assumed constant, the electromotive force of the cell is described by the Nernst equation.

In practice however, the electrochemical system never exibits this ideal behaviour.

Deviations from the Nernst equation sometimes become significant and it is therefore necessary to take account of additional contributions to the electromotive force by interfering ions.

The Nicolsky-Eisenman equation, which is an extension of the Nerst equation, provide a satisfactory semiempirical approach to describing the system, namely:

$$EMF = E_o + S \log(a_i + \sum_i^n {}_j K_{ij}(a_j)^{z_i/z_j}$$

where $k_{ij}$ is the selectivity factor.

Again, $E_o = E^o{}_i + E_R + E_D$

where:

$E^o{}_i$ is a constant potential difference including the surrounding potential differences which arise between the filling solution and the membrane,

$E_R$ is a constant potential difference depending on the potential difference between the metal conductor and the filling solution of the indicator electrode (ion-selective) and the potential difference between the metal

conductor and the standard electrolyte in the reference electrode; $E_R$ is independent of variations in the composition of the sample,

$E_D$ is the interliquid potential difference generated between the standard electrolyte and the solution to be examined.

The sum of $E^o{}_i$ and $E_R$ contains all the contributions which remain independent of the sample composition, $E_D$ is variable and depends on the sample, it being generated at the interface between the standard electrolyte (salt bridge) and the solution to be measured, and can sometimes vary significantly.

Obviously, $E_D$ is added to the potential difference at the membrane, and this contribution must be minimized by suitable choice of the standard electrolyte (the reference junction most commonly used is saturated KCl - 4,2M at 25 °C, or 3M).

Again, the electromotive force of an electrochemical cell with an ion-selective electrode and liquid junction depend on the logarithm of the ion activity, which itself depends on the ion concentration in terms of the stoichiometric quantity of the substance. For ionic strenghts > 0.01M, such as found in biological matrices, the concentration cannot be a substitute for activity.

The activity coefficients which relate activity to concentration determined by solutions in mixed electrolytes containing for example physiologically important ions and proteins would much more adequately describe the conditions in biological liquids.

However this approach unfortunately encounters considerable difficulties in that the instrument which determines the value of the activity coefficient by direct potentiometry would have to be the ion-selective electrodes themselves.

Activity scales for pH measurement in physiological solutions of hydrogen ion activity have been successfully introduced (R.G. Bates, C. Vega: Standard for pH measurement in isotonic saline media of ionic strength I = 0.16; Analytical Chemistry vol. 50, No. 9, August 1978).

The subject of activity coefficients is closely associated with calibration problems and the experimental arrangement of the measuring cell.

According to the present invention it is proposed to improve standardization solutions (calibration and control) for analyzers using direct potentiometry with ion-selective electrodes, by providing them with compensation obtained by adding supporting or active salts or electrolytes, which approximate the value of the activity coefficient and reduce the differences originating from the interliquid potential between the reference standard and the sample.

Expressed in terms of correction factor, the effect can be indicated schematically by the following ratios:

$$\frac{\gamma_i \text{ (sample solution)}}{\gamma_i \text{ (standard solution)}} : \text{ for the activity coeficient}$$

and

$$\frac{E_j \text{ (sample solution)}}{E_j \text{ (standard solution)}} : \text{ for interliquid potential}$$

Accurate calibrations for pathological fluids are obtained with so-called compensated standardizations in which the aforesaid ratios approximate to 1.

In practice, the compensation is obtained with salts which act on the interliquid potential and on the activity coefficient which, in the contingency herein described, is obtained by adding NaCl, KCl and $CaCl_2$ in suitable stoichiometric quantity to obtain best compensation for each ion measured, and obtain the data according to the reference system ($Na^+$ and $K^+$ by flame photometry and Ca by atomic absorption).

The conventional "buffer pair" (pH 7.384 and pH 6.840 at 37 °C ) for standardizing current pH/ISE analyzers, obtained by suitably mixing phosphates of alkali metals in accordance with the NBS formulation, is unsuitable for the simultaneous standardization of sodium, potassium, calcium and chloride for reasons

discussed below in connection with table 1 and 2.

The present invention provides a standardization system for use in a clinical chemistry analyzer consisting of two buffer solutions having ion selective electrode means to allow simultaneous or discrete determination of pH, sodium, potassium, calcium and chloride in whole blood, plasma or serum, said standardization system comprising two buffer solutions, the second buffer solution having the non-zero values of pH and of Na, K, Cl and Ca ions each different from the non-zero values of pH and of Na, K, Cl and Ca ions of the first buffer solution, the first and the second buffer solutions each containing a Good's Buffer selected between TES, HEPES, MOPS and MOPSO with pH-values of the buffers within a range of 6,0 to 8,0 in addition to defined amounts of sodium, potassium, cloride and calcium ions.

Advantageously the first buffer solution contains the buffer pair HEPES/NaHEPES at a concentration of 40 to 60 mmol/liter and the second buffer solution contains the buffer pair MOPS/NaMOPS at the concentration of 40 to 60 mmol/liter.

The invention proposes the formulation of combined-composition buffers for electrolytes and pH, suitable for simultaneous standardization of analyzers using the potentiometric method with ion-selective electrodes.

The chosen criteria for formulating these standardization solution according to the invention were:

- buffer solutions having a pH in the range 6-8 at 37°C;
- solutions having an ionic strength as similar as possible to that of whole blood;
- solutions in which the variation of the ions concerned reflected that in the blood;
- solutions which as far as possible did not contain ions normally absent from the blood;
- solutions prepared with substances having a high state of purity and easily available commercially;
- solutions in which an ionic concentration difference gives a significant difference in terms of $\Delta E$ ($\Delta E > 8$ mV), while still reflecting the ion variation in the blood.

Preferred standardization system are ones meeting all of these criteria, but the standardization systems are suitable in accordance with the invention even if some of the criteria are not met entirely.

The literature data were taken as the starting point for their formulation (R.G. Bates, C. Vega, cited article).

Once prepared, the buffer solutions were subjected to a series of tests regarding two aspects of specific interest, namely 1) pH, and 2) concentration of electrolytes ($Na^+$, $K^+$, etc.).

The pH of the buffer solutions was measured at 37°C with a pH glass electrode as the indicator electrode and a calomel in saturated KCl reference electrode with an open junction.

The calibration was done with buffers obtained from NBS standard reference products, namely pH 6.840 at 37°C, 0.025 molar in $KH_2PO_4$ and $NA_2HPO_4$; pH 7.384 at 37°C, 0.008695 molar $KH_2PO_4$ and 0.03043 molar $NA_2HPO_4$.

The sodium and potassium composition was checked by flame emission photometry and calcium by atomic absorption.

By this method a check was obtained on the total quantity of sodium, potassium and calcium for comparison with the result obtained at the electrodes (ISE) both at 37°C and 25°C.

The electrodes had previously been standardized with pure NaCl, KCl and $CaCl_2$ solutions.

Table 1 shows an example of the results obtained for pH 7,4 at 37°C, IS 0,16 with phosphate buffer to NBS formulation:

TABLE 1

| | Flame photometry moles/l | ISE 25°C moles/l | ISE 37°C moles/l |
|---|---|---|---|
| $Na^+$ | 154 | 135 | 140 |
| $K^+$ | 5.4 | 4.4 | 4.8 |

Table 2 shows an example of the results obtained for pH 6,8 at 37°C, IS 0,16 M with phosphate buffer to NBS formulation:

TABLE 2

| | Flame photometry moles/l | ISE 25 ° C moles/l | ISE 37 ° C moles/l |
|---|---|---|---|
| $Na^+$<br>$K^+$ | 100<br>8 | 85<br>6.1 | 90<br>6.3 |

The results show that calibrating the ion-selective electrodes for sodium and potassium with alkaline phosphate buffers of ionic strength 0.16 leads to a different measurement if compared with non-buffered solutions and gives results less than those of flame photometry or in any event less than the stoichiometric quantity present in the solution.

TABLE 3

| pH | Δ%ISE/FF 25 ° C | | Δ%ISE/FF 37 ° C | |
|---|---|---|---|---|
| | $Na^+$ | $K^+$ | $Na^+$ | $K^+$ |
| 7.4 | 88 | 82 | 91 | 88 |
| 6.8 | 85 | 76 | 90 | 79 |

Table 3 shows that this effect can be partly attributed to the contribution of the liquid junction potential originated by the buffer obtained by the phosphate species. In this respect, the differences indicate that the sodium ions and potassium ions undergo ionic association phenomena with the phosphate buffer.

The extent of this effect was in all cases such as to make it clearly desirable to obtain a buffer pair which behaved towards the electrolytes as a "primary" standardization solution, with behaviour similar to an aqueous NaCl, KCl and $CaCl_2$ solution the pH of which is however adequate for the standardization of an analytical system for determining pH, sodium, potassium, calcium and chloride in the blood and undiluted serum.

For this reason, according to the invention attention was fixed on some derivatives in the form of ethane and propane sulphonic acid (ie Good's Buffer: TES, HEPES, MOPS and MOPSO) with pH values such as to allow buffers to be prepared within the stated pH range (6-8)

HEPES 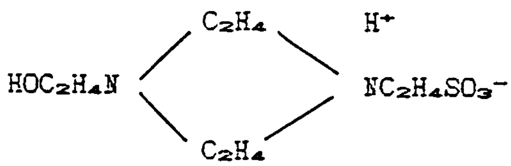 pka at 25°C 7.565

N(2-hydroxyethyl)piperazine - N.ethanesulphonic acid

TES 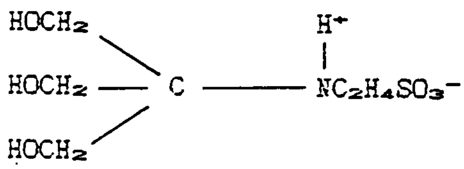

N(trishydroxymethyl) methylamino ethanesulphonic acid

MOPS 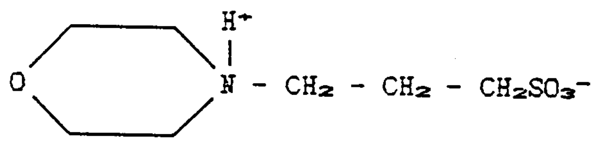 pka at 20°C 7.2

3(N.morpholino) propanesuphonic acid

MOPSO 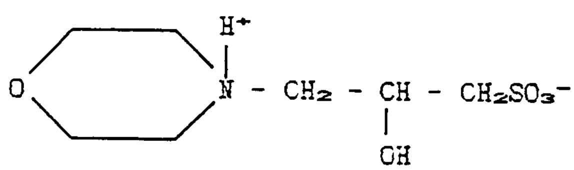 pka at 20°C 6.95

3(N.morpholino)-2-hydroxypropanesuphonic acid

In attaining the objects of the invention it was considered an essential preliminary to solve the problem of compensation of the aspect related to the incomplete dissociation.

The analysis of this condition was extended by using as reference a buffer system based on the pair trishydroxymethylamino-methane (Tris) - Tris HCl, pH 7.5 at 25 ° C, identified as among those which during experimental work had shown little binding capacity towards either sodium, potassium or calcium.

As an example, Table 4 shows the variation in the electromotive force of the Ca electrode against SCE cell in solutions of the given concentration.

## TABLE 4

**Data on the potential of the calcium electrode in the respective solutions at 25°C and 37°C.**

| | Sol. 1 | Sol. 2 | Sol. 3 |
|---|---|---|---|
| 25°C | +42.3 mV | +42.5 mV | +42.6 mV |
| 37°C | +42.8 mV | +42.8 mV | +42.8 mV |

Sol. 1 1 mM Tris/Tris HCl pH 7.2 at 37°C (7.5 at 25°C)
160 mM NaCl
1 mM $CaCl_2$

Sol. 2 10 mM Tris/Tris HCl
152 mM NaCl
1 mM $CaCl_2$

Sol. 3 100 mM Tris/Tris HCl
80 mM NaCl
1 mM $CaCl_2$

Table 5 shows the sodium and potassium variation for ISE against SCE at various Tris/Tris HC1 concentrations.

TABLE 5

Data on electrode potentials against SCE for $NA^+$ and $K^+$ at temperature of 5°C and 37°C

Sodium electrode

| 1 | 50 | 100 | Conc. in Tris/Tris HCl m.moles/l |
|---|---|---|---|
| -11.1 | -10.9 | -10.9 | Electrode pot. (mV) at 25°C |
| -13.3 | -12.88 | -12.7 | Electrode pot. (mV) at 37°C |

Potassium electrode

| | | | |
|---|---|---|---|
| 1 | 50 | 100 | Conc. in Tris/Tris HCl m.moles/l |
| +56.7 | +56.9 | +56.9 | Electrode pot. (mV) at 25°C |
| +47.7 | +47.6 | +47.6 | Electrode pot. (mV) at 37°C |

Sol. 1 1 mM Tris/Tris HCl pH 7.2 at 37°C

140 mM NaCl

5 mM KCl

Sol. 2 50 mM Tris/Tris HCl

140 mM NaCl

5 mM KCl

Sol. 3 100 mM Tris/Tris HCl

140 mM NaCl

5 mM KCl

Using this preliminary analysis an investigation was made of the buffer pair (among those reported) which gave the best buffer effect and the least complexing (binding) effect towards sodium, potassium and calcium.

The standardization system used for sodium and potassium involved the use of a Tris/Tris HCl 1 mM buffer of pH 7.5 at 25 ° C, of 140 mM $Na^+$ and 5 mM $K^+$, and of 100 mM $Na^+$ and 3mM $K^+$.

Table 6 shows the effect of the concentration of the HEPES/NaHEPES buffer pair (pH 7.4 at 37 ° C) on the sodium and potassium determination.

TABLE 6

Data on concentrations in mmoles of Na and K for various HEPES/NaHEPES concentrations at 25°C and 37°C.

a) Sodium

| Sol A | Sol B | Sol C | Sol D | Sol E | HEPES/NaHEPES conc. |
|---|---|---|---|---|---|
| 1 | 10 | 25 | 50 | 100 | |
| 140 | 140 | 138 | 137 | 133 | Na conc. mmoles/l at 25°C |
| 139 | 138 | 136 | 135 | 130 | Na conc. mmoles/l at 37°C |
| -0.7 | -1.4 | -3 | -3.6 | -7.1 | ΔC% against ref. 37°C |

b) Potassium

| Sol A | Sol B | Sol C | Sol D | Sol E | HEPES/NaHEPES conc. |
|---|---|---|---|---|---|
| 1 | 10 | 25 | 50 | 100 | |
| 5 | 5 | 5 | 4.9 | 4.8 | K conc. mmoles/l at 25°C |
| 4.9 | 4.9 | 4.9 | 4.8 | 4.6 | K conc. mmoles/l at 37°C |
| -2 | -2 | -2 | -4 | -8 | ΔC% against ref. 37°C |

c) Calcium

| Sol A | Sol B | Sol C | Sol D | Sol E | HEPES/NaHEPES conc. |
|---|---|---|---|---|---|
| | 0.98 | 0.96 | 0.96 | 0.87 | $Ca^{++}$ conc. mmoles/l at 25°C |
| | 1 | 0.95 | 0.97 | 0.89 | $Ca^{++}$ conc. mmoles/l at 37°C |
| | | 5 | 3 | 11 | ΔC% against ref. 37°C |

For up to 50 mM HEPES/NaHEPES, preferably for 40 to 60 mM concentration of this buffer pair, the effect due to ion complexing by the buffer can be excluded and the differences be attributed to the interliquid potential established at the saturated KCl junction.

To confirm this assumption the calcium concentration at the calcium electrode was read in a series of solutions of constant sodium ion concentration using the sodium electrode as the reference electrode.

The calibration was done with 1 and 3 mM $Ca^{++}$ in Tris/Tris HCl 0.01 M buffer, pH 7.5 at 25°C in 140 mM $Na^{+}$.

The results are shown in Table 7. The measurement solutions differ in HEPES/NaHEPES concentration.

TABLE 7

| HEPES/NaHEPES mM | 10 | 25 | 50 | 100 |
|---|---|---|---|---|
| Calcium observed at 25 ° C against $Na^+$ electrode as reference | 1.02 | 1.01 | 1.00 | 1.00 |
| Calcium observed at 25 ° C against SCE | 1.00 | 0.95 | 0.92 | 0.90 |

These experimental results obtained by keeping the ionic strength constant confirm that the contribution provided by the interliquid potential (against ref. SCE) is determining.

The greater accuracy difference (about double concentration) deriving from it for calcium compared with sodium and potassium is attributable to the double charge of the first ion compared with the others.

Table 8 shows the results of a similar experiment conducted for sodium and potassium in MOPS/NaMOPS buffer at different concentrations.

TABLE 8

Data on concentrations in mmoles of Na and K for various MOPS/NaMOPS concentratins at 25°C and 37°C

a) Sodium

| Sol 1 | Sol 2 | Sol 3 | Sol 4 | Sol 5 | |
|---|---|---|---|---|---|
| 1 | 10 | 25 | 50 | 100 | MOPS/NaMOPS conc. |
| 101 | 99 | 98 | 99 | 92 | Na conc. mmoles/l at 25°C |
| 100 | 99 | 98 | 99 | 93 | Na conc. mmoles/l at 37°C |

b) Potassium

| Sol 1 | Sol 2 | Sol 3 | Sol 4 | Sol 5 | |
|---|---|---|---|---|---|
| 1 | 10 | 25 | 50 | 100 | MOPS/NaMOPS conc. |
| 3 | 3.2 | 2.9 | 3 | 2.9 | K conc. mmoles/l at 25°C |
| 3 | 3.05 | 2.9 | 2.9 | 2.85 | K conc. mmoles/l at 37°C |

The situation is not as simple as in the case of HEPES/NaHEPES.

Again in this case, considering a concentration of 50 mmoles/l of MOPS/NaMOPS, preferably for 40 to 60 mM concentration of this buffer, the effect on sodium and potassium can be considered comparable with that expected.

Both for MOPS/NaMOPS and HEPES/NaHEPES, according to the invention experiments were carried out on the preparations having a concentration of 50 mmoles/l by effecting the compensations which the experimental results had shown necessary for the electrolytes in terms of ionic strength and liquid junction.

| | CORRECTION TO BE MADE IN TERMS OF STOICHIOMETRIC CONCENTRATION COMPARED WITH THAT EXPECTED | | |
|---|---|---|---|
| | $Na^+$ | $K^+$ | $Ca^{++}$ |
| HEPES/NaHEPES 50 mmoles/l | +4% | +4% | +8% |
| MOPS/NaMOPS 50 mmoles/l | +1% | +1% | +2% |

The next experiment was to obtain for the two described buffer solutions the required pH value, namely pH 7.384 (HEPES/NaHEPES) and pH 6.840 (MOPS/NaMOPS) at 37°C, by suitably modifying their proportions while maintaining their total molarity (50 mmoles/litre) constant.

The hydrogen ion activity was determined on a system with a glass electrode and open junction using calomel in saturated KCl at 37°C. Standardization was obtained with phosphate buffers of NBS formulation (7.384 and 6.840 at 37°C).

The buffer stoichiometric compositions are given in Table 9.

## TABLE 9

A) HEPES/NaHEPES buffer — pH 7.384 at 37°C

| | mmoles/l |
|---|---|
| NaHEPES | 27.5 |
| HEPES | 22.36 |
| NaCl | 119 |
| KCl | 5.2 |
| $CaCl_2$ | 1.8 |

+ 20 mg/l of phenylmercuric nitrate ($C_6H_5HgOHC_6H_5HgNO_3$)

B) MOPS/NaMOPS buffer — pH 6.840 at 37°C

| | moles/l |
|---|---|
| NaMOPS | 23.25 |
| MOPS | 28.76 |
| NaCl | 77.75 |
| KCl | 2.02 |
| $CaCl_2$ | 3.06 |

+ 20 mg/l of phenylmercuric nitrate

Table 10 illustrated one example of the control method used to verify the behaviour of the solutions according to the invention.

## TABLE 10

a) HEPES/NaHEPES buffer

| | pH meter 37°C open liquid junction | pH meter 37°C dialysis membrane (hemogas analyzer 1306) |
|---|---|---|
| pH | 7.390 | 7.384 |
| | flame photom. | ISE |
| $Na^+$ | 146 | 140 |
| $K^+$ | 5.2 | 5.0 |
| | atomic. abs | |
| $Ca^{++}$ | 1.08 | 1.00 |

b) MOPS/NaMOPS buffer

| | pH meter 37°C open liquid junction | pH meter 37°C dialysis membrane (hemogas analyzer 1306) |
|---|---|---|
| pH | 6.850 | 6.840 |
| | flame photom. | ISE |
| $Na^+$ | 101 | 100 |
| $K^+$ | 2.02 | 2.00 |
| | Ass. At | |
| $Ca^{++}$ | 3.06 | 3.00 |

The salts used for these standardization systems are stable, commercially obtainable and do not interfere with the determination at the electrodes. The phenylmercuric nitrate acts as a preservative and prevents any bacteria or mould growth in the proposed solutions without requiring sterilization, and does not interfere with or damage the electrodes for pH, sodium, potassium and calcium at the proposed concentrations.

The proposed standardization solutions reduce the error originating from the activity coefficient and from the presence of the liquid junction potential, to provide results for samples of normal blood (for total protein, cholesterol, triglyceride and plasma water contents), serum or plasma, which are in accordance with those determined for sodium and potassium by indirect methods (such as flame photometry or dil ISE) or by comparison methods for pH and ionized calcium conducted with existing commercial instrumentation.

The result of this invention is therefore to attain and offer a standardization system formulation which is suitable for and particularly dedicated to the simultaneous determination of pH and electrolytes in the blood, serum or plasma, with a chemical analyzer using ion-selective electrodes and which compares with instrumentation for the discrete determination of these quantities already described heretofore.

## Claims

1. A standardization system for use in a clinical chemistry analyzer consisting of two buffer solutions having ion selective electrode means to allow simultaneous or discrete determination of pH, sodium, potassium, calcium and chloride in whole blood, plasma or serum, said standardization system comprising two buffer solutions, the second buffer solution having the non-zero values of pH and of Na, K, Cl and Ca ions each different from the non-zero values of pH and of Na, K, Cl and Ca ions of the first buffer solution, the first and the second buffer solutions each containing a Good's Buffer selected between TES, HEPES, MOPS and MOPSO with pH-values of the buffers within a range of 6,0 to 8,0 in addition to defined amounts of sodium, potassium, cloride and calcium ions.

2. The standardization system of claim 1 wherein the first buffer solution contains the buffer pair HEPES/NaHEPES at a concentration of 40 to 60 mmol/liter.

3. The standardization system of claim 1 or 2 wherein the second buffer solution contains the buffer pair MOPS/NaMOPS at a concentration of 40 to 60 mmol/liter.

4. The standardization system of any previous claim wherein the first buffer solution has a pH of a defined value not more than 7.45 nor less than 7,35 and the second buffer solution has a pH of a defined value not more than 6.90 nor less than 6.80.

5. The standardization system of any previous claim wherein the first buffer solution contains substantially the following total amounts of the following elements as ions:

| | |
|---|---|
| Na | 146.5 mmol/liter |
| K | 5.2 mmol/liter |
| Ca | 1,8 mmol/liter |
| Cl | 128 mmol/liter. |

6. The standardization system of any previous claim wherein the second buffer solution contains substantially the following amounts of the following elements as ions:

| | |
|---|---|
| Na | 101 mmol/liter |
| K | 2 mmol/liter |
| Ca | 3 mmol/liter |
| Cl | 83 mmol/liter |

7. The standardization system of any previous claim wherein each of the first and second buffer solutions contains phenylmercuric nitrate at a concentration of 5 to 20 mg/liter.

8. The standardization system of any previous claim wherein the ion selective electrode means of the analyzer performs simultaneous determinations of pH, $Na^+$, $K^+$, $Ca^{++}$ and $Cl^-$ by the potentials of five ion specific electrodes versus a standard silver/silver chloride or calomel reference electrode having a KCl fill solution.

9. A standardization system according to claim 1 in which the simultaneous or discrete determination of pH, sodium, potassium, calcium and chloride in whole blood, plasma or serum, is performed via ISE under salt bridge conditions of at least 3mM KCl but preferably saturated at 37° C (Ag/AgCl or calomel as reference electrode) and the first standard implemented is pH 7,4 (± 0,05) obtained by the buffer

pair HEPES/NaHEPES of 40-60 mmoles/l and preferably about 50 mmoles/l plus the addition of Na, K and Ca ions, substantially in the following percentages:

| | mmoles/l |
|---|---|
| NaHEPES | 27,5 |
| HEPES | 22,36 |
| NaCl | 119 |
| KCl | 5,2 |
| $CaCl_2$ | 1,8 |

**10.** A standardization system as claimed in claim 9 which contains a plurality of ions in concentrations different from the first standard in order to implement the second calibration point at PH 6.85 (± 0.05) obtained by the buffer pair MOPS/NaMOPS of 40-60 mmoles/l and preferably about 50 mmoles/l, plus the addition of $Na^+$, $K^+$ and $Ca^{++}$ ions, substantially in the following percentages:

| | mmoles/l |
|---|---|
| NaMOPS | 23.25 |
| MOPS | 28.76 |
| NaCl | 77.75 |
| Kcl | 2.02 |
| $CaCl_2$ | 3.06 |

**11.** A standardization system as claimed in claim 10 wherein the sodium, potassium, calcium and chloride ions are present in the solution in a stoichiometric concentration such as to compensate for and reduce the error arising from the activity coefficient differences for each ion to be determined with respect to the unknown solution, and to minimize the contribution provided by the existence of the interliquid potential observed for the buffer formulation at the proposed HEPES/NaHEPES and MOPS/NaMOPS concentration of 40-60 mmoles/l and preferably about 50 mmoles/l.

**12.** A standardization system as claimed in claim 11 wherein the calibration for the first point (pH 7.4) for sodium, potassium, calcium and chloride via ISE is obtained respectively at 5 mM (± 0.5 mM), 1 mM (± 0.25 mM), 120 mM (± 5mM) and 140 mM (± 10 mM) by adding each ion in stoichiometric quantity such as to compensate for all contributions due to ionic strength and liquid junction.

**13.** A standardization system as claimed in claim 11 wherein the solution which implements the second calibration point contains the buffer pair MOPS/NaMOPS at 50 mmoles/l and pH 6.85 (± 0.5) and also allows calibration for sodium, potassium, calcium and chloride by forming a $\Delta mV \geqq 3$ mV (never less) for each electrode (against AgCl or calomel in saturated KCl or 3M KCl as reference) with respect to the elctromotive force supplied in the standard provided by the HEPES/NaHEPES solution.

**14.** A standardization system as claimed in claim 9, containing a solution preserving and stabilizing agent which does not alter the buffer characteristics and which preferably consists of phenylmercuric nitrate in a concentration of between 5 and 20 mg/l.

## Patentansprüche

**1.** Ein Normungssystem zur Verwendung an einem Analysator für die klinische Chemie, bestehend aus zwei Pufferlösungen mit ionenselektiven Elektrodenmitteln, um die gleichzeitige oder die getrennte Bestimmung des pH, Natriums, Kaliums, Kalziums und Chlorids im Vollblut, Plasma oder Serum, wobei das Normungssystem zwei Pufferlösungen enthält, wobei die zweite Pufferlösung Werte verschieden von Null des pH und der Na-, K-, Cl- und Ca- Ionen jeweils unterschiedlich von den von Null verschiedenen Werten des pH und der Na-, K-, Cl- und Ca- Ionen der ersten Pufferlösung aufweist, wobei die erste und die zweite Pufferlösung jeweils einen unter TES, HEPES, MOPS und MOPSO

gewählten Good - Puffer mit den pH-Werten der Puffer innerhalb des Bereiches 6,0 bis 8,0 zuzüglich der festgelegten Mengen von Natrium-, Kalium-, Chlorid- und Kalziumionen enthält.

**2.** Normungssystem gemäß Anspruch 1, bei dem die erste Pufferlösung das Pufferpaar HEPES/NaHEPES in einer Konzentration von 40 bis 60 mmol/l enthält.

**3.** Normungssystem nach Anspruch 1 oder 2, bei dem die zweite Pufferlösung das Pufferpaar MOPS/NaMOPS in einer Kozentration von 40 bis 60 mmol/l enthält.

**4.** Normungssystem nach einem der vorstehenden Ansprüche, bei dem die erste Pufferlösung einen zwischen nicht mehr als 7,45 und nicht weniger als 7,35 festgelegten pH-Wert und die zweite Pufferlösung einen zwischen nicht mehr als 6,90 und nicht weniger als 6,80 festgelegten pH-Wert besitzt.

**5.** Normungssystem nach einem der vortehenden Ansprüche, bei dem die erste Pufferlösung im wesentlichen die folgenden Gesamtmengen der folgenden Elemente als Ionen enthält:

| | |
|---|---|
| Na | 146.5 mmol/l |
| K | 5.2 mmol/l |
| Ca | 1.8 mmol/l |
| Cl | 128 mmol/l |

**6.** Normungssystem nach einem der vorstehenden Ansprüche, bei dem die zweite Pufferlösung im wesentlichen die folgenden Mengen der folgenden Elemente als Ionen enthält:

| | |
|---|---|
| Na | 101 mmol/l |
| K | 2 mmol/l |
| Ca | 3 mmol/l |
| Cl | 83 mmol/l |

**7.** Normungssystem nach einem der vorstehenden Ansprüche, bei dem sowohl die erste als auch die zweite Pufferlösung Phenilquecksilbernitrat in einer Konzentration von 5 bis 20 mg/l enthält.

**8.** Normungssystem nach einem der vorstehenden Ansprüche, bei dem die ionenselektiven Elektrodenmittel des Analysators gleichzeitige Bestimmungen des pH, Na+, K+, Ca++ und Cl- mittels der Potentiale der 5 ionenspezifischen Elektroden gegenüber einer Standard-Bezugselektrode in Silber/Silberchlorid oder Kalomel mit einer KCl Füllösung vollziehen.

**9.** Ein Normungssystem nach Anspruch 1, bei dem die gleichzeitige oder getrennte Bestimmung des pH, Natriums, Kaliums, Kalziums und Chlorids im Vollblut, Plasma oder Serum, auf dem Wege ISE unter Salzbrückenbedingungen von mindestens 3 mM KCl, jedoch vorzugsweise gesättigt bei 37°C (Ag/AgCl oder Kalomel als Bezugselektrode) durchgeführt wird und die erste durchgeführte Normung pH 7,4 (± 0,05) ist, das durch das Pufferpaar HEPES/NaHEPES 40-60 mmol/l und vorzugsweise bei 50 mmol/l plus dem Zusatz von Na-, K- und Ca-Ionen, wesentlich mit den folgenden Anteilen erhalten wird:

| | mmol/l |
|---|---|
| NaHEPES | 27,5 |
| HEPES | 22,36 |
| NaCl | 119 |
| KCl | 5,2 |
| $CaC1_2$ | 1,8 |

10. Ein Normungssystem wie unter Anspruch 9 beansprucht, das eine Vielzahl von Ionen in von der ersten Normung verschiedenen Konzentrationen enthält, um den zweiten Eichpunkt bei pH 6,85 (± 0,05) zu verwirklichen, der durch das erste Pufferpaar MOPS/NaMOPS von 40-60 mmol/l erhalten wird, und vorzugsweise bei 50 mmol/l mit Zusatz von $NA^+$, $K^+$, $Ca^{++}$ Ionen im wesentlichen mit den folgenden Anteilen:

| | mmol/l |
|---|---|
| NaMOPS | 23,25 |
| MOPS | 28,76 |
| NaCl | 77,75 |
| KCl | 2,02 |
| $CaCl_2$ | 3,06 |

11. Ein Normungssystem wie unter Anspruch 10 beansprucht, bei dem die Ionen Natrium, Kalium, Chlorid und Kalzium in der Lösung in einer stöchiometrischen Konzentration derart anwesend sind, daß der von den unterschiedlichen Aktivitätskoeffizienten für jedes gegenüber der unbekannten Lösung zu bestimmenden Ions verursachte Fehler ausgeglichen und herabgesetzt wird und um den Beitrag auf ein Mindestmaß herabzusetzen, der durch die Anwesenheit des Potentials zwischen den Flüssigkeiten geliefert wird, und der bezüglich der Formulierung des Puffers bei der vorgeschlagenen Konzentration des HEPES/NaHEPES und MOPS/NaMOPS 40-60 von mmol/l und vorzugsweise bei 50 mmol/l beobachtet wird.

12. Ein Normungssystem wie unter Anspruch 11 beansprucht, bei dem die Eichung des ersten Punktes (pH 7,4) für Natrium, Kalium, Kalzium und Chlorid auf dem Wege ISE jeweils bei 5 mM (± 0,5mM), 1mM (± 0,25mM), 120 mM (± 5 mM) und 140 mM (± 10 mM) durch Zusatz eines jeden Ions in einer derartigen stöchiometrischen Menge erhalten wird, daß die gesamten der ionischen Kraft und der flüssigen Verbindung zuzuschreibenden Beiträge ausgeglichen werden.

13. Ein Normungsystem wie unter Anspruch 11 beansprucht, bei dem die den zweiten Eichpunkt durchführende Lösung das Pufferpaar MOPS/NaMOPS bei 50 mmol/l und pH 6,85 (± 0,5) enthält und auch die Eichung von Natrium, Kalium und Clorid erlaubt, wobei ein $\Delta$ mV ≥3mV (niemals darunter) für jede Elektrode (gegenüber AgCl oder Kalomel als Bezug aus KCl oder 3M KCl) gegenüber der im durch die Lösung HEPES/NaHEPES erhaltenen Standard gelieferten elektromotorischen Kraft erlaubt.

14. Ein Normungssystem wie unter Anspruch 9 beansprucht, beinhaltend einen konservierenden und stabilisierenden Wirkstoff, der die Merkmale des Puffers nicht verändert und vorzugsweise aus Phenilquecksilbernitrat in einer Konzentration von 5 bis 20 mg/l besteht.

**Revendications**

1. Système d'étalonnage à utiliser dans un analyseur pour chimie clinique comportant deux solutions tampons ayant des moyens à électrode de sélection de ions pour permettre la détermination simultanée ou distincte de pH, sodium, potassium, calcium et chlorure dans le sang entier, dans le plasma et dans le sérum, ledit système d'étalonnage comprenant deux solutions tampons, la seconde solution tampon ayant les valeurs non nulles de pH et de ions Na, K, Cl et Ca chacune différente des valeurs non nulles de pH et de ions Na, K, Cl et Ca de la première solution tampon, les première et seconde solutions tampons contenant chacune un tampon Good choisi entre TES, HEPES, MOPS et MOPSO avec des valeurs de pH des tampons dans la gamme de 6 à 8 en plus de quantités définies de ions sodium, potassium, chlorure et calcium.

2. Système d'étalonnage selon la revendication 1, caractérisé en ce que la première solution tampon contient la paire tampon HEPES/NaHEPES suivant une concentration de 40 à 60 mmol/litre.

3. Système d'étalonnage selon la revendication 1 ou 2, caractérisé en ce que la seconde solution tampon contient la paire tampon MOPS/NaMOPS suivant une concentration de 40 à 60 mmol/litre.

4. Système d'étalonnage selon l'une quelconque des revendications précédentes, caractérisé en ce que la première solution tampon a un pH de valeur définie non supérieure à 7,45 et non inférieure à 7,35 et la seconde solution tampon a un pH de valeur définie non supérieure à 6,90 et non inférieure à 6,80.

5. Système d'étalonnage selon l'une quelconque des revendications précédentes, caractérisé en ce que la première solution tampon contient essentiellement les quantités suivantes des éléments qui suivent en tant que ions:

| | |
|---|---|
| Na | 146,5 mmol/litre |
| K | 5,2 mmol/litre |
| Ca | 1,8 mmol/litre |
| Cl | 128 mmol/litre |

6. Système d'étalonnage selon l'une quelconque des revendications précédentes, caractérisé en ce que la seconde solution tampon contient essentiellement les quantités suivantes des éléments qui suivent en tant que ions:

| | |
|---|---|
| Na | 101 mmol/litre |
| K | 2 mmol/litre |
| Ca | 3 mmol/litre |
| Cl | 83 mmol/litre |

7. Système d'étalonnage selon l'une quelconque des revendications précédentes, caractérisé en ce que chacune des première et seconde solutions tampons contient du nitrate phénylmercurique dans une concentration de 5 à 20 mg/litre.

8. Système d'étalonnage selon l'une quelconque des revendications précédentes, caractérisé en ce que les moyens à électrode de l'analyseur accomplissent les déterminations simultanées de pH, Na+, K+, Ca++ et Cl-moyennant les potentiels d'électrodes spécifiques de cinq ions contre un électrode de référence standard d'argent/chlorure d'argent ou calomel ayant une solution de saturation de KCl.

9. Système d'étalonnage selon la revendication 1, caractérisé en ce que la determination simultanée ou distincte de pH, sodium, potassium, calcium et chlorure dans le sang entier, plasma ou sérum est accomplie via ISE en conditions de pont salin d'au moins 3 mM KCl mais de préférence saturé à 37° C (Ag/AgCl ou calomel en tant qu'électrode de référence) et le premier étalon réalise pH 7,4 (± 0,05) obtenu par la paire tampon HEPES/NaHEPES de 40-60 mmoles/l et de préférence environ 50 mmoles/l plus l'adjonction de ions Na, K et Ca, essentiellement dans les pourcentages qui suivent:

| | mmoles/1 |
|---|---|
| NaHEPES | 27,5 |
| HEPES | 22,36 |
| NaCl | 119 |
| KCl | 5,2 |
| $CaCl_2$ | 1,8 |

10. Système d'étalonnage selon la revendication 9, caractérisé en ce qu'il contient une plurarité de ions dans des concentrations différentes par rapport au premier étalon dans le but de réaliser le second point de calibrage à pH 6,85 (± 0,05) obtenu par la paire MOPS/NaMOPS de 40-60 mmoles/l et de préférence environ 50 mmoles/l plus l'adjonction de ions Na+, K+ et Ca++, essentiellement dans les pourcentages qui suivent:

| | mmoles/l |
|---|---|
| NaMOPS | 23,25 |
| MOPS | 28,76 |
| NaCl | 77,75 |
| KCl | 2,02 |
| $CaCl_2$ | 3,06 |

**11.** Système d'étalonnage selon la revendication 10, caractérisé en ce que les ions sodium, potassium, calcium et chlorure sont présents dans la solution suivant une concentration stoechiométrique en mesure de compenser et réduire l'erreur qui résulte des différences de coefficient d'activité pour chaque ion à déterminer par rapport à la solution inconnue et de minimiser la contribution résultant de l'existence du potential interliquide observé pour la formulation tampon à la concentration proposée de HEPES/NaHEPES et MOPS/NaMOPS de 40-60 mmoles/l, de préférence approximativement 50 mmoles/l.

**12.** Système d'étalonnage selon la revendication 11, caractérisé en ce que le calibrage du premier point (pH 7,4) pour sodium, potassium, calcium et chlorure via ISE est obtenu respectivement a 5 mM (± 0,5 mM), 1 mM (± 0,25 mM), 120 mM (± 5 mM) et 140 mM (± 10 mM) par l'adjonction de chaque ion en quantité stoechiométrique de manière à compenser toutes les contributions dues à la force ionique et à la jonction liquide.

**13.** Système d'étalonnage selon la revendication 11, caractérisé en ce que la solution qui réalise le second point de calibrage contient une paire tampon MOPS/NaMOPS à 50 mmoles/l et pH 6,85 (± 0,5) et permet également le calibrage de sodium, potassium, calcium et chlorure moyennant la formation d'un $\Delta$ mV $\geqq$ 3 mV (jamais inférieur) pour chaque électrode (contre AgCl ou calomel en KCl saturé ou 3M KCl en tant que référence) par rapport à la force électromotrice fournie dans l'étalon prévu par la solution HEPES/NaHEPES.

**14.** Système d'étalonnage selon la revendication 9, caractérisé en ce qu'il contient un agent de conservation et stabilisation de la solution qui ne modifie pas les caractéristiques du tampon et qui de préférence se compose de nitrure phénylmercurique dans une concentration allant de 5 à 20 mg/l.